# EUROPÄISCHE PATENTSCHRIFT

(11) **EP 1 398 074 B1**
(45) Veröffentlichungstag und Bekanntmachung des Hinweises auf die Patenterteilung: **29.10.2008**
(21) Anmeldenummer: 02020211.5
(22) Anmeldetag: 10.09.2002
(51) Int. Cl.: B01J 13/16, B01J 13/20, A61K 8/11, D06M 23/12

(54) **Polyamidmikrokapseln (III)**
Polyamide Microcapsules (III)
Microcapsules de polyamide (III)

(43) Veröffentlichungstag der Anmeldung: 17.03.2004
(73) Patentinhaber: Cognis IP Management GmbH, 40589 Düsseldorf (DE)
(72) Erfinder: Viladot Petit, Josep-Lluis, Dr., 08018 Barcelona (ES); Tacies, Anna, 08034 Barcelona (ES)

(56) Entgegenhaltungen:
- US-A- 4 518 547
- US-A- 4 534 783
- US-B1- 6 242 405
- US-B1- 6 359 031

## Beschreibung

### Gebiet der Erfindung

Die Erfindung befindet sich auf dem Gebiet der verkapselten Wirkstoffe und betrifft neue Mikrokapseln mit Polyamidhüllen, ein Verfahren zu deren Herstellung sowie deren Verwendung in einer Reihe von Einsatzgebieten.

### Stand der Technik

Unter dem Begriff "Mikrokapsel" werden vom Fachmann sphärische Aggregate mit einem Durchmesser im Bereich von etwa 0,0001 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) *: Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Chitosanmikrokapseln und Verfahren zu ihrer Herstellung sind Gegenstand früherer Patenanmeldungen der Patentanmelderin [WO 01/01926, WO 01/01927, WO 01/01928, WO 01/01929]. Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, können beispielsweise erhalten werden, indem man
(a1) aus Gelbildnern, Chitosanen und Wirkstoffen eine Matrix zubereitet,
(a2) gegebenenfalls die Matrix in einer Ölphase dispergiert,
(a3) die dispergierte Matrix mit wässrigen Lösungen anionischer Polymere behandelt und gegebenenfalls dabei die Ölphase entfernt.
oder
(b1) aus Gelbildnem, anionischen Polymeren und Wirkstoffen eine Matrix zubereitet,
(b2) gegebenenfalls die Matrix in einer Ölphase dispergiert,
(b3) die dispergierte Matrix mit wässrigen Chitosanlösungen behandelt und gegebenenfalls dabei die Ölphase entfernt.
oder
(c1) wässrige Wirkstoffzubereitungen mit Ölkörpern in Gegenwart von Emulgatoren zu O/W-Emulsionen verarbeitet,
(c2) die so erhaltenen Emulsionen mit wässrigen Lösungen anionischer Polymere behandelt,
(c3) die so erhaltene Matrix mit wässrigen Chitosanlösungen in Kontakt bringt und
(c4) die so erhaltenen Verkapselungsprodukte von der wässrigen Phase abtrennt.

Von Nachteil ist jedoch, dass die Mikrokapseln des Stands der Technik gegenüber aggressiven Medien nur sehr bedingt stabil sind. Kapseln des oben genannten Typs lösen sich beispielsweise in Hypochloritlösungen oder Wasserstoffperoxid schlicht auf, so dass sie mit diesen Stoffen nicht in Kontakt gebracht werden können. Andererseits ist es gerade für den amerikanischen und den südeuropäischen Raum, in dem Einsatz von solchen Mitteln für die Reinigung harter Oberflächen sehr verbreitet ist, wünschenswert, Mikrokapseln zur Verfügung stellen zu können, die sich in diesen Medien als hinreichend stabil erweisen und die darin enthaltenen Wirkstoffe erst nach Zuführung mechanischer Energie freisetzen.

Die Aufgabe der vorliegenden Erfindung hat folglich darin bestanden, neue Mikrokapseln mit mittleren Durchmessern im Bereich von 0,0001 bis 0,1 mm zur Verfügung zu stellen, die in hypochlorit- bzw. wasserstoffperoxidhaltigen Lösungen stabil sind, d.h. sich über einen Zeitraum von wenigstens 4 Wochen nicht auflösen oder den sie enthaltenen Wirkstoff anders freisetzen.

Polyamidmikrokapseln werden in US-B-6 242 405 beschreiben.

### Beschreibung der Erfindung

Gegenstand der Erfindung sind neue Polyamidmikrokapseln mit einem mittleren Durchmesser im Bereich von 0,0001 bis 0,1 mm, die dadurch erhältlich sind, dass man
(a) eine wässrige Phase herstellt, in der (a1) Diamine und/oder Triamine, (a2) Tenside, (a3) Heteropolysaccharide oder Polymere sowie (a4) Wirkstoffe gelöst sind,
(b) eine Ölphase herstellt, in der (b1) Dicarbonsäurechloride gelöst sind, und
(c) die beiden Phasen unter Ausbildung einer W/O-Emulsion in Kontakt bringt, so dass an den Grenzflächen eine Polykondensationsreaktion unter Bildung von Polyamidmikrokapseln stattfindet.

Überraschenderweise wurde gefunden, dass sich gerade Polyamidkapseln durch eine besondere Beständigkeit in Hypochloritlaugen und peroxidischen Mitteln auszeichnen. Die so erhaltenen sphärischen Gebilde haben einen mittleren Durchmesser von maximal 0,1 mm und sind über einen Zeitraum von wenigstens 4 Wochen in gängigen hypochlorithaltigen Haushaltsreinigern beständig.

### Verfahren

Ein weiterer Gegenstand der Erfindung betrifft ein Verfahren zur Herstellung von Polyamidmikrokapseln mit einem mittleren Durchmesser im Bereich von 0,0001 bis 0,1 mm, bei dem man
(a) eine wässrige Phase herstellt, in der (a1) Diamine und/oder Triamine, (a2) Tenside (a3) Heteropolysaccharide oder Polymere sowie (a4) Wirkstoffe gelöst sind,
(b) eine Ölphase herstellt, in der (b1) Dicarbonsäurechloride gelöst sind, und
(c) die beiden Phasen unter Ausbildung einer W/O-Emulsion in Kontakt bringt, so dass an den Grenzflächen eine Polykondensationsreaktion unter Bildung von Polyamidmikrokapseln stattfindet.

### Diamine und Triamine

In einem ersten Schritt des erfindungsgemäßen Verfahrens wird eine wässrige Zubereitung hergestellt, welche als Komponente (a1) das für die Polykondensation benötigte Di- und/oder Triamin enthält. Die Auswahl dieser Komponente ist wenig kritisch, demnach kommen grundsätzlich alle aliphatischen oder aromatischen Di- oder Triamine in Betracht, die über eine hinreichende Wasserlöslichkeit verfügen. Besonders bevorzugt ist indes das für Nylon-6,6 bekannte Hexamethylendiamin. Der Einsatz von Triaminen bietet infolge der bei der Polykondensation stattfindenden Vernetzung den Vorteil, dass die Mikrokapseln stabiler werden. Die wässrige Phase enthält die Komponente (a1) üblicherweise in Mengen von 0,1 bis 5, vorzugsweise 0,5 bis 2 Gew.-%.

### Tenside

Als Komponente (a2) kommen sowohl nichtionische als auch kationische, amphotere oder zwitterionische oberflächenaktive, zur - W/O - Emulsionsbildung befähigte Verbindungen in Betracht. Vorzugsweise werden jedoch anionische Tenside eingesetzt. Typische Beispiele für geeigente anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Vorzugsweise werden typische W/O-Emulgatoren wie z.B. Alkylbenzolsulfonate, Alkylsulfate, Seifen, Alkansulfonate, Olefinsulfonate, Methylestersulfonate sowie deren Gemische eingesetzt.
➢Alkylbenzolsulfonate

Bevorzugte Alkylbenzolsulfonate folgen der Formel **(I)**,
**R¹-Ph-SO₃X** (I)
in der R¹ für einen verzweigten, vorzugsweise jedoch linearen Alkylrest mit 10 bis 18 Kohlenstoffatomen, Ph für einen Phenylrest und X für ein Alkali- und/oder Erdalkalimetall, Ammonium, Alkylammonium, Alkanolammonium oder Glucammonium steht. Insbesondere von diesen geeignet sind Dodecylbenzolsulfonate, Tetradecylbenzolsulfonate, Hexadecylbenzolsulfonate sowie deren technische Gemische in Form der Natriumsalze.
➢ Alkyl- und/oder Alkenylsulfate

Unter Alkyl- und/oder Alkenylsulfaten, die auch häufig als Fettalkoholsulfate bezeichnet werden, sind die Sulfatierungsprodukte primärer und/oder sekundärer Alkohole zu verstehen, die vorzugsweise der Formel **(II)** folgen,

**R²O-SO₃X** (II)

in der R² für einen linearen oder verzweigten, aliphatischen Alkyl- und/oder Alkenylrest mit 6 bis 22, vorzugsweise 12 bis 18 Kohlenstoffatomen und X für ein Alkali- und/oder Erdalkalimetall, Ammonium, Alkylammonium, Alkanolammonium oder Glucammonium steht. Typische Beispiele für Alkylsulfate, die im Sinne der Erfindung Anwendung finden können, sind die Sulfatierungsprodukte von Capronalkohol, Caprylalkohol, Caprinalkohol, 2-Ethylhexylalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technischen Gemischen, die durch Hochdruckhydrierung technischer Methylesterfraktionen oder Aldehyden aus der Roelen'schen Oxosynthese erhalten werden. Die Sulfatierungsprodukte können vorzugsweise in Form ihrer Alkalisalze und insbesondere ihrer Natriumsalze eingesetzt werden. Besonders bevorzugt sind Alkylsulfate auf Basis von C_{16/18}-Talg-Fettalkoholen bzw. pflanzliche Fettalkohole vergleichbarer C-Kettenverteilung in Form ihrer Natriumsalze. Im Falle von verzweigten primären Alkoholen handelt es sich um Oxoalkohole, wie sie z.B. durch Umsetzung von Kohlenmonoxid und Wasserstoff an alpha-ständige Olefine nach dem Shop-Verfahren zugänglich sind. Solche Alkoholmischungen sind im Handel unter dem Handelsnamen Dobanol® oder Neodol® erhältlich. Geeignete Alkoholmischungen sind Dobanol 91®, 23®, 25®, 45®. Eine weitere Möglichkeit sind Oxoalkohole, wie sie nach dem klassischen O-xoprozess der Enichema bzw. der Condea durch Anlagerung von Kohlenmonoxid und Wasserstoff an Olefine erhalten werden. Bei diesen Alkoholmischungen handelt es sich um eine Mischung aus stark verzweigten Alkoholen. Solche Alkoholmischungen sind im Handel unter dem Handelsnamen Lial® erhältlich. Geeignete Alkoholmischungen sind Lial 91®, 111®, 123®, 125®, 145®.

➢ Seifen

Unter Seifen sind weiterhin Fettsäuresalze der Formel **(III)** zu verstehen,

**R³CO-OX** (III)

in der R³CO für einen linearen oder verzweigten, gesättigten oder ungesättigten Acylrest mit 6 bis 22 und vorzugsweise 12 bis 18 Kohlenstoffatomen und wiederum X für Alkali- und/oder Erdalkali, Ammonium, Alkylammonium oder Alkanolammonium steht. Typische Beispiele sind die Natrium-, Kalium-, Magnesium-, Ammonium- und Triethanolammoniumsalze der Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, E-laeostearinsäure, Arachinsäure, Gadoleinsäure, Behensäure und Erucasäure sowie deren technische Mischungen. Vorzugsweise werden Kokos- oder Palmkernfettsäure in Form ihrer Natrium- oder Kaliumsalze eingesetzt.

Die wässrige Phase enthält die Komponente (a2) üblicherweise in Mengen von 0,1 bis 5, vorzugsweise 0,5 bis 2 Gew.-%.

### Heteropolysaccharide und Polymere

Die Heteropolysaccharide bzw. Polymere, die die fakultative Komponente (a3) bilden, dienen zur leichteren Bildung einer Matrix.

➢ Heteropolysaccaride

Als Heteropolysaccharide kommen vorzugsweise Agarosen in Frage, welche in Form des aus Rotalgen zu gewinnenden Agar-Agar auch zusammen mit bis zu 30 Gew.-% nichtgelbildenden Agaropektinen vorliegen können. Hauptbestandteil der Agarosen sind lineare Polysaccharide aus D-Galaktose und 3,6-Anhydro-L-galaktose, die alternierend β-1,3-und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen.

➢ Anionische Polymere

Als anionische Polymere eignen sich vorzugsweise Salze der Alginsäure. Bei der Alginsäure handelt es sich um ein Gemisch carboxylgruppenhaltiger Polysaccharide mit folgendem idealisierten Monomerbaustein:

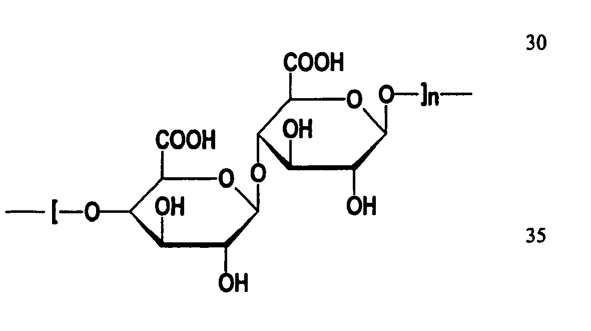

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.
➢ Chitosane

Als Polymere kommen des weiteren Chitosane in Frage. Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

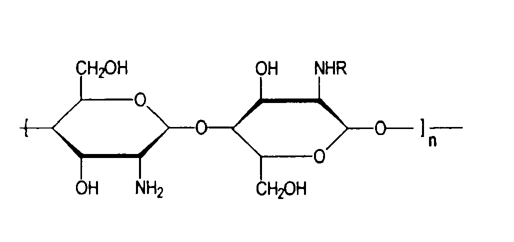

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt. Anstelle der Chitosane können auch beliebige andere kationische Polymere eingesetzt werden.

Die wässrige Phase enthält die Komponente (a3) üblicherweise in Mengen von 0,1 bis 5, vorzugsweise 0,5 bis 2 Gew.-%.

### Wirkstoffe

In einer bevorzugten Ausführungsform der vorliegenden Erfindung enthalten die Mikrokapseln in ihrer inneren wässrigen Phase - vorzugsweise wasserlösliche bzw. wasserdispergierbare - Wirkstoffe (Komponente a4), deren Auswahl weitgehend unkritisch ist und sich ausschließlich nach der Aufgabenstellung richtet. Typische Beispiele sind Enzyme, biogene Wirkstoffe, Antioxidantien, UV-Filter, Parfümöle und Aromen, Farbstoffe bzw. Farbpigmente sowie vollständige kosmetische oder pharmazeutische Emulsionen. Im Hinblick auf Verwendungen in Gegenwart von Hypochloriten oder Peroxiden ist der Einsatz von Enzymen, Farbund Duftstoffen bevorzugt, die ansonsten in dieser Umgebung chemisch instabil wären. Nichtsdestotrotz können die Kapseln natürlich auch in wässriger Umgebung, also beispielsweise in der Kosmetik oder der Textiltechnik eingesetzt werden. Die wässrige Phase enthält die Komponente (a4) üblicherweise in Mengen von 0,1 bis 25, vorzugsweise 1 bis 10 und insbesondere 2 bis 5Gew.-%. Typische Beispiele für geeignete Wirkstoffgruppen werden im folgenden erläutert:

➢ Enzyme

Als Enzyme kommen insbesondere solche aus der Klasse der Hydrolasen, wie der Proteasen, Esterasen, Lipasen bzw. lipolytisch wirkenden Enzyme, Amylasen, Cellulasen bzw. andere Glykosylhydrolasen und Gemische der genannten Enzyme in Frage. Alle diese Hydrolasen tragen in der Wäsche zur Entfernung von Verfleckungen, wie protein-, fettoder stärkehaltigen Verfleckungen, und Vergrauungen bei. Cellulasen und andere Glykosylhydrolasen können durch das Entfernen von Pilling und Mikrofibrillen zur Farberhaltung und zur Erhöhung der Weichheit des Textils beitragen. Zur Bleiche bzw. zur Hemmung der Farbübertragung können auch Oxidoreduktasen eingesetzt werden. Besonders gut geeignet sind aus Bakterienstämmen oder Pilzen, wie Bacillus subtilis, Bacillus licheniformis, Streptomyces griseus und Humicola insolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease- und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder O-xidasen haben sich in einigen Fällen als geeignet erwiesen. Zu den geeigneten Amylasen zählen insbesondere α-Amylasen, Iso-Amylasen, Pullulanasen und Pektinasen. Als Cellulasen werden vorzugsweise Cellobiohydrolasen, Endoglucanasen und β-Glucosidasen, die auch Cellobiasen genannt werden, bzw. Mischungen aus diesen eingesetzt. Da sich die verschiedenen Cellulase-Typen durch ihre CMCase- und Avicelase-Aktivitäten unterscheiden, können durch gezielte Mischungen der Cellulasen die gewünschten Aktivitäten eingestellt werden.

➢ Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Dihydroxyaceton, Arbutin, Ferulasäure, Kojisäure, Cumarinsäure und Ascorbinsäure (Vitamin C), Aminosäuren, Ceramide, Pseudoceramide, essentielle Öle, Pflanzenextrakte, wie z.B. Prunusextrakt, Bambaranussextrakt und Vitaminkomplexe zu verstehen.

➢ Antioxidantien

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesterylund Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, U-bichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

➢ UV-Lichtschutzfilter

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher beschrieben;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)-benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
➢ Propan-1,3-dione, wie z.B. 1-(4-tert.Butylphenyl)-3-(4`methoxyphenyl)propan-1,3-dion;
➢ Ketotricyclo(5.2.1.0)decan-Derivate.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4`-tert.Butylphenyl)-3-(4`-methoxyphenyl)propan-1,3-dion, 4-tert.-Butyl-4'-methoxydibenzoylmethan (Parsol® 1789), 1-Phenyl-3-(4`-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

➢Antischuppenwirkstoffe

Typische Beispiele für Antischuppenwirkstoffe sind Pirocton, Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl)r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfelteer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat.

➢ Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, I-raldein gamma, Phenylessigsäure, Geranylacetat, Benzylacetat, Rosenoxid, Romilllat, I-rotyl und Floramat allein oder in Mischungen, eingesetzt. Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

➢ Farbstoffe und Farbpigmente

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein.

### Ölphase

Im zweiten Verfahrensschritt wird die Ölphase hergestellt, welche die zweite Kondensationskomponente, nämlich das Dicarbonsäurechlorid (Komponente b1) enthält. Auch hier ist die Auswahl der Dicarbonsäurekomponente wenig kritisch, im Hinblick auf die Herstellung von Nylon-6,6-Kapseln empfiehlt sich natürlich wieder Sebacinsäurechlorid. Alternativ können aber auch längerkettige Dicarbonsäuren, wie die bei der Fermentation von entsprechenden Paraffinen anfallende 1,18-Hexadecandicarbonsäure bzw. deren Chlorid eingesetzt werden. Auch die Auswahl des Öls ist wenig kritisch. In der Regel wird man Mineralöl ("Weißöl") bzw. Paraffinöl einsetzen, ebenfalls geeignet sind auch Siliconöle oder Esteröle sowie alle übrigen gängigen kosmetischen Ölkörper. Die Ölphase enthält die Dicarbonsäurechloride üblicherweise in Mengen von 0,1 bis 5 und vorzugsweise 2 bis 3 Gew.-%.

### Emulsionsbildung und Polykondensation, Härtung und Aufarbeitung

Im dritten Schritt findet die eigentliche Phasengrenzflächenpolykondensation statt. Hierzu werden die wässrige Phase und die Ölphase unter starker Scherung mit einander vermischt, wobei eine W/O-Emulsion erhalten wird. Typischerweise setzt man die beiden Phasen im Gewichtsverhältnis 3 : 1 bis 1 : 3 und vorzugsweise etwa 2 : 1 bis 1 : 2 ein. An den Grenzflächen der Wassertröpfchen zu der sie umgebenden Ölphase findet eine Polykondensation zwischen den Diaminen und den Dicarbonsäurechloriden statt und es bilden sich sphärische Gebilde mit einer Polyamidhülle und einer wässrigen inneren Phase, in der die Wirkstoffe enthalten sind. Diese Mikrokapseln werden vorsichtig abfiltriert und mit Wasser oder verdünnter Tensidlösung gewaschen, um anhaftende Reste der Ölphase zu entfernen.

### Gewerbliche Anwendbarkeit

Ein weiterer Gegenstand der Erfindung betrifft die Verwendung der neuen Polyamidmikrokapseln zur Herstellung von hypochlorithaltigen Zubereitungen, speziell von Mitteln zur Reinigung harter Oberflächen, welche die Kapseln in Mengen von 1 bis 10 Gew.-% - bezogen auf die Mittel - enthalten können. Gleichwohl eignen sich die Mikrokapseln auch für andere Anwendungszwecke, beispielsweise zur Herstellung von kosmetischen Zubereitungen und zur Ausrüstung von textilen Flächengebilden.

### Beispiele

### Beispiel 1

In einem 1-1-Dreihalskolben mit Rührer und Tropftrichter wurden bei Raumtemperatur 0,3 g Hexamethylendiamin, 0,5 g Natriumlaurylsulfat, 0,1 g Agar Agar, 0,05 g Phenonip und 0,05 g Indanthrenblau RS (C.I. 69800) vorgelegt und mit destilliertem Wasser auf 100 ml aufgefüllt. Zu der Lösung wurde unter starkem Rühren innerhalb von 3 min 100 g einer 5 Gew.-%igen Lösung von Sebacinsäurechlorid in Paraffinöl getropft. Nach etwa 30 min war die Polykondensation abgeschlossen und die erhaltenen Polyamidmikrokapseln wurden vorsichtig über einem Büchnerfilter von der Ölphase abgetrennt und mit einer 3 Gew.-% Natriumlaurylsulfatlösung gewaschen, bis keine Ölreste mehr zu erkennen waren. Der mittlere Durchmesser der Kapseln betrug 0,001 mm.

### Beispiel 2

In einem 1-1-Dreihalskolben mit Rührer und Tropftrichter wurden bei Raumtemperatur 0,3 g Hexamethylendiamin, 0,5 g Natriumdodecylsulfonat, 0,2 g Calciumalginat, 0,05 g Phenonip und 0,05 g Krapplack (C.I.58000) vorgelegt und mit destilliertem Wasser auf 100 ml aufgefüllt. Zu der Lösung wurde unter starkem Rühren innerhalb von 3 min 100 g einer 5 Gew.-%igen Lösung von Sebacinsäurechlorid in Paraffinöl getropft. Nach etwa 30 min war die Polykondensation abgeschlossen und die erhaltenen Polyamidmikrokapseln wurden vorsichtig über einem Büchnerfilter von der Ölphase abgetrennt und mit einer 3 Gew.-% Natriumdodecylsulfonatlösung gewaschen, bis keine Ölreste mehr zu erkennen waren. Der mittlere Durchmesser der Kapseln betrug 0,001 mm.

### Beispiel 3

In einem 1-1-Dreihalskolben mit Rührer und Tropftrichter wurden bei Raumtemperatur 0,3 g Hexamethylendiamin, 0,5 g Palmkernfettsäure-Kaliumsalz, 0,1 g Chitosan, 0,05 g Phenonip und 0,1 g Retinol vorgelegt und mit destilliertem Wasser auf 100 ml aufgefüllt. Zu der Lösung wurde unter starkem Rühren innerhalb von 3 min 100 g einer 3 Gew.-%igen Lösung von 1,18-Hexadecandicarbonsäurechlorid in Paraffinöl getropft. Nach etwa 30 min war die Polykondensation abgeschlossen und die erhaltenen Polyamidmikrokapseln wurden vorsichtig über einem Büchnerfilter von der Ölphase abgetrennt und mit einer 3 Gew.-% Seifenlösung gewaschen, bis keine Ölreste mehr zu erkennen waren. Der mittlere Durchmesser der Kapseln betrug 0,001 mm.

### Beispiel 4

Die gemäß Beispiele 1 bis 3 erhaltenen Polyamidmikrokapseln wurden in eine 5 Gew.-%ige Natriumhydroxidlösung eingebracht und dort über einen Zeitraum von 4 Wochen bei 30 °C gelagert. Alle Systeme erwiesen sich dabei als stabil.

## Patentansprüche

1. Polyamidmikrokapseln mit einem mittleren Durchmesser im Bereich von 0,0001 bis 0,1 mm, **dadurch** erhältlich, dass man
(a) eine wässrige Phase herstellt, in der
(a1) Diamine und/oder Triamine,
(a2) Tenside,
(a3) Heteropolysaccharide oder Polymere sowie
(a4) Wirkstoffe gelöst sind,
(b) eine Ölphase herstellt, in der (b1) Dicarbonsäurechloride gelöst sind, und
(c) die beiden Phasen unter Ausbildung einer W/O-Emulsion in Kontakt bringt, so dass an den Grenzflächen eine Polykondensationsreaktion unter Bildung von Polyamidmikrokapseln stattfindet.

2. Verfahren zur Herstellung von Polyamidmikrokapseln mit einem mittleren Durchmesser im Bereich von 0,0001 bis 0,1 mm, bei dem man
(a) eine wässrige Phase herstellt, in der
(a1) Diamine und/oder Triamine,
(a2) Tenside,
(a3) Heteropolysaccharide oder Polymere sowie
(a4) Wirkstoffe gelöst sind,
(b) eine Ölphase herstellt, in der (b1) Dicarbonsäurechloride gelöst sind,
(c) die beiden Phasen unter Ausbildung einer W/O-Emulsion in Kontakt bringt, so dass an den Grenzflächen eine Polykondensationsreaktion unter Bildung von Polyamidmikrokapseln stattfindet,
(d) die Polyamidmikrokapseln abfiltriert und wäscht,
(e) die so aufgereinigten Polyamidmikrokapseln mit einer Calciumsalzlösung behandelt und dabei härtet, und schließlich
(f) die gehärteten Polyamidmikrokapseln erneut abfiltriert, wäscht und trocknet.

3. Verfahren nach Anspruch 2, **dadurch gekennzeichnet, dass** man als Diamin (Komponente a1) Hexamethylendiamin einsetzt.

4. Verfahren nach den Ansprüchen 2 und/oder 3, **dadurch gekennzeichnet, dass** man als Komponente (a2) anionische Tenside einsetzt, die ausgewählt sind aus der Gruppe, die gebildet wird von Seifen, Alkylbenzolsulfonaten, Alkansulfonaten, Olefinsulfonaten, Alkylethersulfonaten, Glycerinethersulfonaten, α-Methylestersulfonaten, Sulfofettsäuren, Alkylsulfaten, Fettalkoholethersulfaten, Glycerinethersulfaten, Hydroxymischethersulfaten, Monoglycerid-(ether)sulfaten, Fettsäureamid(ether)sulfaten, Mono- und Dialkylsulfosuccinaten, Mono- und Dialkylsulfosuccinamaten, Sulfotriglyceriden, Amidseifen, Ethercarbonsäuren und deren Salzen, Fettsäureisethionaten, Fettsäuresarcosinaten, Fettsäuretauriden, N-Acylaminosäuren, Alkyloligoglucosidsulfaten, Proteinfettsäurekondensaten und Alkyl(ether)phosphaten.

5. Verfahren nach mindestens einem der Ansprüche 2 bis 4, **dadurch gekennzeichnet, dass** man als Komponente (a3) Heteropolysaccharide oder Polymere einsetzt, die ausgewählt sind aus der Gruppe, die gebildet wird von Agarosen, Alginaten und Chitosanen.

6. Verfahren nach mindestens einem der Ansprüche 2 bis 5, **dadurch gekennzeichnet, dass** man wässrige Phasen einsetzt, die die Komponenten (a1), (a2) und (a3) in Mengen von jeweils 0,1 bis 5 Gew.-% enthalten.

7. Verfahren nach mindestens einem der Ansprüche 2 bis 6, **dadurch gekennzeichnet, dass** man als Komponente (a4) Wirkstoffe einsetzt, die ausgewählt sind aus der Gruppe, die gebildet wird von Enzymen, biogenen Wirkstoffen, Antioxidantien, UV-Filtem, Parfümölen und Aromen, Farbstoffen bzw. Farbpigmenten sowie vollständige kosmetischen oder pharmazeutischen Emulsionen.

8. Verfahren nach mindestens einem der Ansprüche 2 bis 7, **dadurch gekennzeichnet, dass** man wässrige Phasen einsetzt, die die Komponente (a4) in Mengen von jeweils 0,1 bis 25 Gew.-% enthalten.

9. Verfahren nach mindestens einem der Ansprüche 2 bis 8, **dadurch gekennzeichnet, dass** man als Komponente (b1) Sebacinsäurechlorid einsetzt.

10. Verfahren nach mindestens einem der Ansprüche 2 bis 9, **dadurch gekennzeichnet, dass** man Ölphasen einsetzt, die die Komponente (b) in Mengen von 0,1 bis 5 Gew.-% enthalten.

11. Verfahren nach mindestens einem der Ansprüche 2 bis 10, **dadurch gekennzeichnet, dass** man die wässrige Phase und die Ölphase im Gewichtsverhältnis 3 : 1 bis 1 : 3 einsetzt.

12. Verwendung von Polyamidmikrokapseln nach Anspruch 1 zur Herstellung von hypochlorithaltigen Zubereitungen.

13. Verwendung von Polyamidmikrokapseln nach Anspruch 1 zur Herstellung von kosmetischen Zubereitungen.

14. Verwendung von Polyamidmikrokapseln nach Anspruch 1 zur Ausrüstung von textilen Flächengebilden.

## Claims

1. Polyamide microcapsules with an average diameter in the range from 0.0001 to 0.1 mm, obtainable by
(a) preparing an aqueous phase in which
(a1) diamines and/or triamines,
(a2) surfactants,
(a3) heteropolysaccharides or polymers, and
(a4) active ingredients are dissolved,
(b) preparing an oil phase in which (b1) dicarboxylic acid chlorides are dissolved, and
(c) bringing the two phases into contact with formation of a W/O emulsion, such that a polycondensation reaction takes place at the interfaces with formation of polyamide microcapsules.

2. Process for the preparation of polyamide microcapsules with an average diameter in the range from 0.0001 to 0.1 mm, in which
(a) an aqueous phase is prepared in which
(a1) diamines and/or triamines,
(a2) surfactants,
(a3) heteropolysaccharides or polymers, and
(a4) active ingredients are dissolved,
(b) an oil phase is prepared in which (b1) dicarboxylic acid chlorides are dissolved,
(c) the two phases are brought into contact with formation of a W/O emulsion, such that a polycondensation reaction takes place at the interfaces with formation of polyamide microcapsules,
(d) the polyamide microcapsules are filtered off and washed,
(e) the polyamide microcapsules purified in this way are treated with a calcium salt solution and hardened in so doing, and finally
(f) the hardened polyamide microcapsules are filtered off again, washed and dried.

3. Process according to Claim 2, **characterized in that** hexamethylenediamine is used as diamine (component al).

4. Process according to Claims 2 and/or 3, **characterized in that** anionic surfactants selected from the group which is formed from soaps, alkylbenzenesulphonates, alkanesulphonates, olefinsulphonates, alkyl ether sulphonates, glycerol ether sulphonates, α-methyl ester sulphonates, sulpho fatty acids, alkylsulphates, fatty alcohol ether sulphates, glycerol ether sulphates, hydroxy mixed ether sulphates, monoglyceride (ether) sulphates, fatty acid amide (ether) sulphates, mono- and dialkyl sulphosuccinates, mono- and dialkyl sulphosuccinamates, sulphotriglycerides, amide soaps, ether carboxylic acids and salts thereof, fatty acid isethionates, fatty acid sarcosinates, fatty acid taurides, N-acylamino acids, alkyl oligoglucoside sulphates, protein fatty acid condensates and alkyl (ether) phosphates are used as component (a2).

5. Process according to at least one of Claims 2 to 4, **characterized in that** heteropolysaccharides or polymers selected from the group which is formed from agaroses, alginates and chitosans are used as component (a3).

6. Process according to at least one of Claims 2 to 5, **characterized in that** aqueous phases which comprise components (a1), (a2) and (a3) in amounts of from in each case 0.1 to 5% by weight are used.

7. Process according to at least one of Claims 2 to 6, **characterized in that** active ingredients selected from the group which is formed from enzymes, biogenic active ingredients, antioxidants, UV filters, perfume oils and aromas, dyes and colour pigments, and also complete cosmetic or pharmaceutical emulsions are used as component (a4).

8. Process according to at least one of Claims 2 to 7, **characterized in that** aqueous phases which comprise component (a4) in amounts of from in each case 0.1 to 25% by weight are used.

9. Process according to at least one of Claims 2 to 8, **characterized in that** sebacoyl chloride is used as component (b1).

10. Process according to at least one of Claims 2 to 9, **characterized in that** oil phases which comprise component (b) in amounts of from 0.1 to 5% by weight are used.

11. Process according to at least one of Claims 2 to 10, **characterized in that** the aqueous phase and the oil phase are used in the weight ratio 3:1 to 1:3.

12. Use of polyamide microcapsules according to Claim 1 for producing hypochlorite-containing preparations.

13. Use of polyamide microcapsules according to Claim 1 for producing cosmetic preparations.

14. Use of polyamide microcapsules according to Claim 1 for the finishing of fabrics.

## Revendications

1. Microcapsules de polyamide d'un diamètre moyen situé dans la plage de 0,0001 mm à 0,1 mm, que l'on peut obtenir :
(a) en préparant une phase aqueuse, dans laquelle sont dissous
(a1) des diamines et/ou des triamines,
(a2) des tensioactifs,
(a3) des hétéropolysaccharides ou des polymères ainsi que
(a4) des substances actives ;
(b) en préparant une phase huileuse, dans laquelle sont dissous des chlorures d'acides dicarboxyliques (bl), et
(c) en mettant en contact les deux phases avec formation d'une émulsion eau-dans-l'huile, de telle sorte qu'il se produise, au niveau des interfaces, une réaction de polycondensation avec formation de microcapsules de polyamide.

2. Procédé de fabrication de microcapsules de polyamide d'un diamètre moyen situé dans la plage de 0,0001 mm à 0,1 mm,
selon lequel :
(a) on prépare une phase aqueuse, dans laquelle sont dissous :
(a1) des diamines et/ou des triamines,
(a2) des tensioactifs,
(a3) des hétéropolysaccharides ou des polymères ainsi que
(a4) des substances actives,
(b) on prépare une phase huileuse, dans laquelle sont dissous des chlorures d'acides dicarboxyliques (b1) et
(c) on met en contact les deux phases avec formation d'une émulsion eau-dans-l'huile, de telle sorte qu'il se produise, au niveau des interfaces, une réaction de polycondensation avec formation de microcapsules de polyamide,
(d) on sépare par filtration et on lave les microcapsules de polyamide,
(e) on traite les microcapsules de polyamide ainsi nettoyées avec une solution de sel de calcium, ce qui les durcit, et enfin
(f) on sépare à nouveau par filtration, on lave et on sèche les microcapsules durcies.

3. Procédé selon la revendication 2,
**caractérisé en ce qu'**
on utilise comme diamine (composant al) l'hexaméthylènediamine.

4. Procédé selon les revendications 2 et/ou 3,
**caractérisé en ce qu'**
on utilise, comme composant (a2), des tensioactifs anioniques qui sont choisis dans le groupe formé par les savons, les alkylbenzènesulfonates, les alcanesulfonates, les oléfinesulfonates, les alkyléthersulfonates, les éthersulfonates de glycérol, les α-méthylestersulfonates, les acides sulfoaliphatiques, les sulfates d'alkyles, les éthersulfates d'alcools gras, les éthersulfates de glycérol, les hydroxyéthersulfates mixtes, les (éther)sulfates de monoglycérides, les (éther)sulfates d'amides d'acides gras, les sulfosuccinates de mono- et di-alkyles, les sulfosuccinamates de mono- et di-alkyles, les sulfotriglycérides, les savons d'amides, les acides éthercarboxyliques et leurs sels, les iséthionates d'acides gras, les sarcosinates d'acides gras, les taurures d'acides gras, les N-acyloaminoacides, les sulfates d'alkyloligoglucosides, les condensés d'acides gras de protéines et les (éther)phosphates d'alkyles.

5. Procédé selon au moins l'une des revendications 2 à 4,
**caractérisé en ce qu'**
on utilise, comme composant (a3), des hétéropolysaccharides ou des polymères, qui sont choisis dans le groupe formé par les agaroses, les alginates et les chitosanes.

6. Procédé selon au moins l'une des revendications 2 à 5,
**caractérisé en ce qu'**
on utilise des phases aqueuses qui contiennent les composants (a1), (a2), et (a3) dans les proportions, à chaque fois, de 0,1 % à 5 % en poids.

7. Procédé selon au moins l'une des revendications 2 à 6,
**caractérisé en ce qu'**
on utilise, comme composant (a4), des substances actives qui sont choisies dans le groupe formé par les enzymes, les substances actives biogènes, les antioxydants, les filtres-UV, les huiles parfumées et les arômes, les colorants ou les pigments colorants ainsi que des émulsions cosmétiques ou pharmaceutiques complètes.

8. Procédé selon au moins l'une des revendications 2 à 7,
**caractérisé en ce qu'**
on utilise des phases aqueuses qui contiennent le composant (a4) dans les proportions, à chaque fois, de 0,1 % à 25 % en poids.

9. Procédé selon au moins l'une des revendications 2 à 8,
**caractérisé en ce qu'**
on utilise, comme composant (b1), le chlorure d'acide sébacique.

10. Procédé selon au moins l'une des revendications 2 à 9,
**caractérisé en ce qu'**
on utilise des phases huileuses qui contiennent le composant (b) dans les proportions de 0,1 % à 5 % en poids.

11. Procédé selon au moins l'une des revendications 2 à 10,
**caractérisé en ce qu'**
on utilise la phase aqueuse et la phase huileuse dans un rapport pondéral de 3/1 1 à 1/3.

12. Utilisation de microcapsules de polyamide selon la revendication 1 pour la production de préparations contenant des hypochlorites.

13. Utilisation de microcapsules de polyamide selon la revendication 1 pour la production de préparations cosmétiques.

14. Utilisation de microcapsules de polyamide selon la revendication 1 pour l'apprêt de produits textiles plats.
